## Europäisches Patentamt
## European Patent Office
## Office européen des brevets

(19)

(11) Veröffentlichungsnummer : **0 075 223**
**B1**

(12) **EUROPÄISCHE PATENTSCHRIFT**

(45) Veröffentlichungstag der Patentschrift :
01.02.89

(51) Int. Cl.⁴ : **G 06 K   7/10**

(21) Anmeldenummer : **82108381.3**

(22) Anmeldetag : **11.09.82**

(54) **Vorrichtung zur optischen Erkennung einer Codierung auf einem Diagnoseteststreifen.**

(30) Priorität : **18.09.81 DE 3137174**

(43) Veröffentlichungstag der Anmeldung :
**30.03.83 Patentblatt 83/13**

(45) Bekanntmachung des Hinweises auf die Patenterteilung : **01.02.89 Patentblatt 89/05**

(84) Benannte Vertragsstaaten :
**AT BE CH DE FR GB IT LI LU NL SE**

(56) Entgegenhaltungen :
**DE—A— 2 436 209**
**US—A— 3 466 451**
**US—A— 3 566 119**
**US—A— 3 907 503**
**US—A— 4 288 701**
**IBM TECHNICAL DISCLOSURE BULLETIN, Band 18, Nr. 3, August 1975, Seite 660, New York, US; F.J.BAELLE: "Photocell operational amplifier with lenticular lens"**
**Die Akte enthält technische Angaben, die nach dem Eingang der Anmeldung eingereicht wurden und die nicht in dieser Patentschrift enthalten sind.**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Ruppender, Uwe**
**Ifflandstrasse 7**
**D-6800 Mannheim 1 (DE)**

EP 0 075 223 B1

Jouve, 18, rue St-Denis, 75001 Paris, France

## Beschreibung

Die vorliegende Erfindung betrifft ein Teststreifenanalysesystem nach dem Oberbegriff des Anspruchs 1.

Ein solches System ist aus der US-PS 3 907 503 bekannt. Die Codierung besteht in diesem Fall aus einem auf einem durchsichtigen Teststreifen angebrachten Codeblock. Die Information steckt bei der bekannten Einrichtung in dem Abstand zwischen diesem Codeblock und dem ersten benachbarten Testfeld auf dem Teststreifen. Zum Messen wird der Teststreifen in zwangsgeführter Weise zwischen einer Lichtquelle und einem Fotoempfänger hindurchgeführt. Das von der Lichtquelle ausgesandte Licht wird von dem Fotoempfänger durch den Teststreifen hindurch aufgenommen. Bei einer gleichmäßigen Bewegung des Teststreifens läßt sich der zeitliche Abstand zwischen der Unterbrechung des Lichtstrahles durch den Codeblock und durch das erste Testfeld als Codierung für eine bestimmte Information verwenden. Im vorliegenden Fall unterscheiden sich verschiedene Teststreifentypen durch die Abstände zwischen Codefeld und erstem Testfeld und durch die Position des Codefeldes auf dem Teststreifen.

Die bekannte Einrichtung ist zwar geeignet, sehr einfache Informationen vom Teststreifen abzulesen. Es wäre jedoch wünschenswert, wenn mehr Information auf dem Teststreifen untergebracht und, beispielsweise beim Einführen des Teststreifens in das Auswertegerät, in dessen elektronische Speicher übertragen werden könnte.

Zur Unterbringung dieser größeren Informationsmenge steht auf dem Teststreifen andererseits nur ein sehr kleiner Raum zu Verfügung. Dies führt zu einer entsprechend hohen Informationsdichte mit entsprechenden Problemen bezüglich Sicherheit und Störungsarmut der Übertragung.

Aufgabe der vorliegenden Erfindung ist es, bei Teststreifen-Analysesystemen die Übertragung einer verhältnismäßig grossen Informationsmenge vom Teststreifen auf das Auswertegerät mit einfachen und kostengünstigen Mitteln zu ermöglichen. Dabei soll eine zuverlässige und störungsarme Übertragung der in der Codierung gespeicherten Information möglich sein.

Zur Lösung dieser Aufgabe werden die Maßnahmen gemäß dem Patentanspruch 1 vorgeschlagen.

Die Verwendung einer Zylinderlinse zum apparativen Lesen von Symbolen ist bekannt. So beschreibt die US-A-3 466 451 eine Vorrichtung zum Lesen von Schriftzeichen, bei der die praktischen Vorteile dieses Linsentyps (kostengünstig, platzsparend) genutzt werden. Ein Hinweis auf die Verwendung in Teststreifen-Analysesystemen oder die Lösung der dabei sich stellenden spezifischen Probleme ist dieser Druckschrift nicht zu entnehmen.

Als Strichcodeschrift eignet sich jede derartige Codierung (sogenannter bar code), die auf einem Teststreifen angebracht werden kann. Sie besteht aus einer Vielzahl zueinander paralleler, in der Regel verschieden dicker und in verschiedenen Abständen auf dem Informationsträger angebrachter Striche. Als Strichcodeschrift hoher Informationsdichte wird eine Codierung verstanden, die mehr als etwa 10 bit vorzugsweise etwa 20 bit Information pro cm enthält.

Die in kurzem Abstand über dem Teststreifen angebrachte Zylinderlinse dient der Fokusierung des Empfänger- bzw. Senderstrahles der Leseeinrichtung auf die Linien des Strichcodes. Dies ließe sich im Prinzip auch mit Hilfe einer sonst für Strichcodeleser üblichen sphärischen Linse erreichen. Diese wäre jedoch für die speziellen Verhältnisse bei einem Teststreifen weniger gut geeignet, da bei der hohen Informationsdichte, wie sie für die Codierung auf Teststreifen notwendig ist, eine sehr scharfe Fokusierung erforderlich ist, durch die selbst kleinste Ungleichmäßigkeiten oder Fehler des Strichcode zu falschen Leseergebnissen führen würden. Derartige kleine Fehler lassen sich jedoch bei den in der Teststreifenproduktion gegebenen Produktionsbedingungen nicht zuverlässig vermeiden.

Für Empfänger und Sender ist eine gemeinsame Zylinderlinse vorgesehen, wobei sich Empfänger und Sender insbesondere, aber nicht notwendigerweise, hinter einem gemeinsamen Spalt befinden. Durch diese Anordnung werden die ohnehin gegebenen Vorteile der Verwendung einer Zylinderlinse noch besser eingesetzt. Die Zylinderlinse hat bevorzugt die gleiche Länge wie die Codestriche. Dadurch wird die gesamte Codestrichlänge gleichzeitig auf den Empfänger abgebildet, d. h. der Empfänger sieht das Integral der etwa in der Fokuslinie der Zylinderlinse liegenden Teststreifenoberfläche über die gesamte Breite der Codestriche. Dadurch läßt sich in idealer Weise eine für die Verhältnisse bei Teststreifen optimale Kombination großer Flankensteilheit der gewonnenen Signale bei gleichzeitiger Fehlersicherheit der Auswertung erreichen.

Diese beiden Forderungen stehen in einem gewissen Gegensatz zueinander. Je schärfer nämlich die Abbildung bei einem Strichcodeleser ist, desto höher wird die Flankensteilheit. Gleichzeitig nimmt jedoch die Gefahr zu, daß kleine Verunreinigungen oder Fehler der Bedruckung des Strichcodeträgers als Signale fehlgedeutet werden und dann beispielsweise zu falschen Meßergebnissen führen.

Für die bei den üblichen diagnostischen Teststreifen gegebenen Verhältnisse muß es möglich sein, noch Codestriche mit weniger als 0,2 mm Breite zuverlässig zu erkennen, wenn man eine größere Informationsmenge, z. B. 60 bit, darauf unterbringen will. Bekannte Leser können aus optischen und konstruktiven Gründen nicht oder nur mit hohem Aufwand die hierzu nötige Auflösung bei gleichzeitiger hinreichender Fehlersicherheit erreichen. Weitere bevorzugte Ausführungsformen der Erfindung werden anhand eines in den Figuren dargestellten Ausführungsbeispieles näher erläutert. Es zeigen :

Fig. 1 eine prinzipielle perspektivische Darstellung der Anordnung der für die Erfindung notwendigen Elemente und

Fig. 2 eine perspektivische Explosionsdarstellung einer praktischen Ausführungsform einer erfindungsgemäßen Vorrichtung.

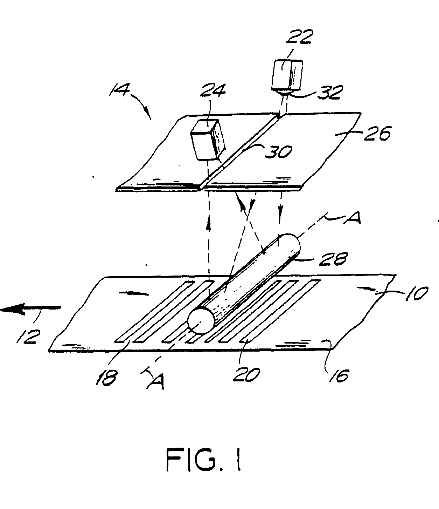

In Fig. 1 erkennt man einen Teil eines Teststreifens 10, der zwangsgeführt in Richtung des Pfeiles 12 unter dem in seiner Gesamtheit mit dem Bezugszeichen 14 versehenen Strichcodeleser hindurch bewegbar ist. Die Zwangsführung sorgt dabei für eine geradlinige Bewegung in Richtung der Teststreifenlängsachse, wobei der Teststreifen 10 so geführt wird, daß die Entfernung zwischen dem Codeleser 14 und der Teststreifenoberfläche 16 praktisch konstant bleibt.

Auf der Teststreifenoberfläche befindet sich eine Strichcodeschrift 18, die aus einzelnen, in ihrer Breite und in ihrem Abstand variierenden Codestrichen 20 besteht. Die Codestriche verlaufen parallel zueinander und senkrecht zur Längsrichtung des Teststreifens und damit zu der durch den Pfeil 12 angedeuteten Bewegungsrichtung.

Die Codeleseeinrichtung 14 besteht aus einem Sender 22, einem Empfänger 24, einer Spaltblende 26 und einer Zylinderlinse 28. Die Zylinderlinse 28 ist im dargestellten bevorzugten Ausführungsbeispiel als Kreiszylinder ausgebildet, dessen Zylinderachse mit dem Bezugszeichen A versehen ist. Zwischen dem Sender 22 und dem Empfänger 24 befindet sich eine nicht dargestellte Blende, durch die eine Einwirkung von Streulicht vom Sender 22 auf den Empfänger 24 verhindert wird. Durch den Sender 22 wird der Spalt 30 in der Spaltblende 26 beleuchtet. Die Zylinderlinse 28 sorgt für eine Abbildung des beleuchteten Spaltes 30 auf die Teststreifenoberfläche 16.

Die gleiche Zylinderlinse 28, deren Länge etwa der der Codestriche 20 entspricht, sorgt auch für die Abbildung der Codestriche auf den Empfänger 24.

In der dargestellten Ausführungsform befinden sich der Sender 22, der Empfänger 24, der Spalt 30 und die Zylinderlinse 28 in einer Ebene, die senkrecht zur Teststreifenoberfläche verläuft. Dadurch wird eine optimale Ausnutzung der Intensität des Lichtsenders 22 und eine weitgehend verzerrungsfreie Abbildung erreicht.

In der dargestellten Ausführungsform ist die Zylinderlinse 28 als Kreiszylinder ausgebildet. Eine derartige Ausführungsform ist besonders kostengünstig herzustellen und leicht einzubauen. Es sind jedoch auch andere Zylinderquerschnitte für den erfindungsgemäßen Zweck geeignet, beispielsweise ein Halbkreis oder ein sonstiger linsenförmiger Querschnitt, der zu einer Bündelung der Strahlen vom Sender 22 bzw. zum Empfänger 24 führt. Derartige Formgebungen haben vielfach bessere Abbildungseigenschaften, jedoch hat sich in der Praxis gezeigt, daß die mit einer Kreiszylinderlinse erzielbare Abbildungsqualität für den vorliegenden Anwendungszweck vollständig ausreichend ist.

Bevorzugt hat die als Kreiszylinder ausgebildete Zylinderlinse 28 einen Durchmesser von weniger als 2 und insbesondere weniger als 1 mm. Durch den Durchmesser wird bei einer Kreiszylinderlinse die Brennweite der Linse bestimmt. Bei einem kleinen Durchmesser ergibt sich somit eine kleine Brennweite und damit ein geringer Abstand zwischen der Zylinderlinse 28 und der Teststreifenoberfläche 16. Dies ist vorteilhaft, weil sich damit eine kleine Bauweise des Codelesers 14 erreichen läßt.

Außerdem ergeben sich bei Verwendung einer kleinen Kreiszylinderlinse für die praktische Anwendung günstige Abbildungsverhältnisse. In einem besonders bevorzugten Ausführungsbeispiel hat die Zylinderlinse einen Durchmesser von 1 mm und eine Brennweite von 0,75 mm. Der Abstand zwischen Zylinderachse A und der Ebene der Spaltblende 26 beträgt 8 mm, wobei der Spalt 0,2 mm breit ist. Bei diesen Verhältnissen wird der Spalt in eine sogenannte « Bildebene » unterhalb der Zylinderlinse scharf abgebildet, die 0,83 mm unterhalb der Achse A der Zylinderlinse liegt. Dennoch hat sich in der Praxis herausgestellt, daß eine besonders zuverlässige Auswertung der Codierung möglich ist, wenn der Abstand zwischen Zylinderlinse 28 und Teststreifenoberfläche 16 etwas geringer ist und z. B. nur 0,7 oder 0,6 mm beträgt. Mit einem solchermaßen gegenüber der Lage der Schärfenebene verkürzten Abstand zwischen Zylinderlinsenachse A und Teststreifenoberfläche 16 wird eine ausreichende Flankensteilheit der Signale bei gleichzeitiger weitgehender Störungsfreiheit und minimaler Verunreinigung der Zylinderlinse erreicht. Bei der beispielhaft angegebenen Dimensionierung lassen sich noch Streifen von 0,04 mm Breite praktisch fehlerfrei erkennen. Wichtig ist jedenfalls, daß sich die Teststreifenoberfläche grundsätzlich etwa in einer solchen Entfernung von der Zylinderlinsenachse A befindet, daß die Fokuslinie der Zylinderlinse näherungsweise in der Teststreifenoberfläche liegt.

Bei einem sehr großen Abstand zwischen dem abzubildenden Spalt 30 und der Linse 28 fällt die Bildebene, in der der Spalt scharf abgebildet wird, weitgehend mit der Fokuslinie der Zylinderlinse zusammen. Praktisch hat sich gezeigt, daß besonders gute Meßergebnisse erreicht werden, wenn der Abstand zwischen der Spaltblende 26 und der Zylinderlinse 28 mindestens etwa zehn mal so groß wie die Brennweite der Linse ist.

Für die Auswertung einer Codierung auf einem diagnostischen Teststreifen hat sich besonders die Verwendung von infrarotem Licht bewährt. Deswegen ist bevorzugt die als Sender verwendete Lichtquelle als Infrarot-Leuchtdiode ausgebildet, während der Empfänger ein infrarotempfindlicher Fototransistor ist. Eine derartige Ausführungsform ist insbesondere für batteriebetriebene Geräte vorteilhaft, weil Infrarot-Leuchtdioden eine besonders hohe Intensität bei geringem Stromverbrauch ermöglichen. Darüber hinaus wurde im Rahmen der vorliegenden Erfindung gefunden, daß die üblichen Teststreifenmaterialien für Infrarotlicht weitgehend durchlässig sind, obwohl sie in normalem Licht undurchsichtig sind. Wenn nun die Codestriche 20 metallische Bestandteile enthalten, wie dies bevorzugt der Fall ist, so ergibt sich ein besonders guter Kontrast zwischen den reflektierenden Codestrichen 20 und dem schwach absorbierenden Material des Teststreifens 10 in infrarotem Licht. Der Sender 22 und der Empfänger 24 tragen bevorzugt integrierte Linsen 32, die das Licht auf den Spalt bündeln.

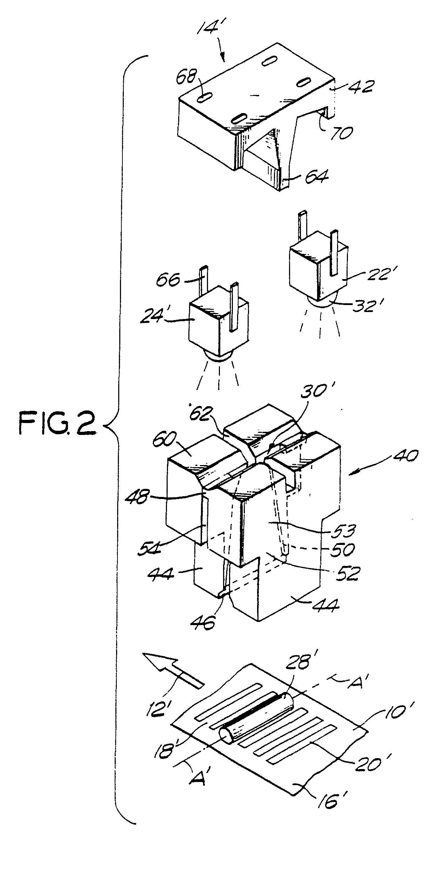

In Fig. 2 ist in einer perspektivischen Explosionsdarstellung eine praktische Ausführungsform der wesentlichen Teile der erfindungsgemäßen Vorrichtung dargestellt. Dabei sind die der Fig. 1 entsprechenden Teile mit dem gleichen Bezugszeichen, versehen mit einem zusätzlichen Strich, bezeichnet. Man erkennt die Zylinderlinse 28' mit ihrer Achse A' über einem Teststreifen 10', der mit einer Strichcodeschrift 18' versehen ist und in Richtung des Pfeiles 12' zwangsgeführt wird.

Darüber erkennt man eine Blenden-Linsen-Einheit 40, den Sender 22' und den Empfänger 24' und eine Sender- und Empfängerhalterung 42. Die Teile sind in vertikaler Richtung auseinandergezogen dargestellt und befinden sich im Gerät im zusammengefügten Zustand in einem vertikalen Schacht für den Codeleser 14', in dem sie genau eingepaßt sind.

Die Blende-Linse-Einheit 40 besteht aus zwei identisch ausgebildeten Formkörpern 44.

Man erkennt am unteren Ende eine Nut 46, deren Form derjenigen der Zylinderlinse 28' entspricht. Oben an dem Formkörper 44 befindet sich ein Steg 48, der, wie aus der Figur zu ersehen, nur auf der einen Seite der dem anderen Formkörper 44 zugewandten Fläche vorgesehen ist. Da beide Formkörper 44 identisch sind, hat der gegenüberliegende Formkörper einen entsprechenden Steg 48, der in der Figur verdeckt ist und auf der entsprechenden Gegenfläche des durchgezogen dargestellten Formkörpers anliegt. Zwischen den beiden Stegen befindet sich bei aneinanderanliegenden Formkörpern 44 ein Spalt, der die Spaltblende 30' bildet. Unter diesem Spalt ist in der Zeichnung eine bogenförmige Ausnehmung 50 zu erkennen, in deren Bereich die von ihr umfaßte Fläche 52 gegenüber der Vorderfläche 54 geringfügig zurückspringt. Im zusammengebauten Zustand ist die Zylinderlinse 28' in die entsprechende Nut 46 eingesetzt und die beiden Formkörper werden in dem nicht dargestellten Codeleserschacht dergestalt gegeneinander gedrückt, daß jeweils die Stege 48 gegen die Vorderflächen 54 des benachbarten Formkörpers 44 anliegen und im unteren Bereich die Zylinderlinse 28' durch die Nuten 46 gehalten ist. Dabei besteht zwischen beiden Formkörpern im Bereich der Vorderflächen 54 ein Abstand, der der Höhe der Stege 48 entspricht und der die Breite des Beleuchtungsspaltes 30' definiert. In einem bevorzugten Ausführungsbeispiel beträgt dieser Abstand 0,2 mm. Im Bereich der Ausnehmung 50 ist die Ausnehmungsfläche 52 beispielsweise 0,2 mm zurückversetzt, so daß sich zwischen den beiden Ausnehmungsflächen ein Lichtschacht 53 von etwa 0,6 mm Breite ergibt, durch den das Licht von dem Sender 22' auf die Zylinderlinse und das reflektierte Licht von der Zylinderlinse auf den Empfänger 24' gelangt.

In der Oberfläche 60 der Formkörper 44 befindet sich eine Nut 62, in die eine Sperrblende 64 der Sender- und Empfängerhalterung 42 eingesetzt werden kann. Durch diese Sperrblende 64 wird eine gegenseitige Beeinflussung von Sender 22' und Empfänger 24' verhindert. Schließlich erkennt man in der Figur noch die Anschlußdrähte 66 die durch entsprechende Bohrungen 68 in der Sender- und Empfängerhalterung 42 zu den entsprechenden elektronischen Einheiten des Gerätes geführt werden können. Die Sender- und Empfängerhalterung 42 hat Aussparungen 70, in die der Sender 22' und der Empfänger 24' formschlüssig eingepaßt und somit zuverlässig gehaltert werden, wenn die Sender- und Empfängerhalterung 42 in die Blende-Linse-Einheit 40 eingesetzt und der daraus resultierende Codeleser 14' in den entsprechenden Schacht des Gerätes eingefügt ist.

**Patentansprüche**

1. Teststreifen-Analysesystem bestehend aus Teststreifen und einem Teststreifen-Auswertegerät, welches eine Vorrichtung zur optischen Erkennung einer Codierung auf dem Teststreifen einschließt, wobei der Teststreifen in dem Auswertegerät relativ zu der Leseeinrichtung für die Codierung zwangsgeführt bewegbar ist und die Leseeinrichtung eine Lichtquelle als Sender und ein lichtempfindliches Element als Empfänger aufweist, dadurch gekennzeichnet, daß

die Teststreifen einen Strichcode mit einer Informationsdichte von mehr als 10 bit pro cm tragen,

der Sender (22) und der Empfänger (24) auf der gleichen Seite des Teststreifens untergebracht sind,

sich zwischen Teststreifen (10) und Sender (22) und zwischen Teststreifen (10) und Empfänger (24) eine gemeinsame Zylinderlinse (28) befindet, deren Zylinderachse (A) im wesentlichen parallel zu der die Codierung tragenden Testfläche (16) und zu den Codestrichen (10) verläuft,

zwischen der Zylinderachse (28) und dem Sender (22) sowie zwischen der Zylinderlinse (28) und dem Empfänger (24) jeweils eine Spaltblende vorhanden ist,

beide Blendenspalte (30) und der Empfänger (24) in der durch die Zylinderachse (A) und den Sender (22) definierten Ebene liegen,

die Entfernung zwischen der Zylinderachse (A) und der Teststreifenfläche (16) näherungsweise so gewählt ist, daß die Fokuslinie der Linse (28) etwa in der Teststreifenoberfläche (16) liegt, und

das Auswertegerät einen Speicher aufweist, in den die auf dem Strichcode codierte Information übertragen wird.

2. Teststreifen-Analysesystem nach Anspruch 1, dadurch gekennzeichnet, daß die beiden Blendenspalten etwa gleich weit von der Zylinderlinse entfernt sind und etwa die gleiche Breite haben.

3. Teststreifen-Analysesystem nach einem der Ansprüche 1 oder 2, dadurch gekennzeichnet, daß der Sender (22), der Empfänger (24) und die Spaltblenden (26, 30) in einer zur Teststreifenoberfläche (16) senkrechten Ebene symmetrisch beidseitig der Teststreifenmittelebene liegen.

4. Teststreifen-Analysesystem nach einem der Ansprüche 1 bis 3, dadurch gekennzeichnet, daß die Zylinderlinse (28) als Kreiszylinder mit einem Durchmesser von weniger als 2 mm ausgebildet ist.

5. Teststreifen-Analysesystem nach Anspruch 4, dadurch gekennzeichnet, daß der Linsendurchmesser maximal 1 mm beträgt.

6. Teststreifen-Analysesystem nach einem der Ansprüche 1 bis 5, dadurch gekennzeichnet, daß der Abstand zwischen der Blende (26) und der Zylinderlinse (28) mindestens etwa zehn mal so groß ist wie die Linsenbrennweite.

7. Teststreifen-Analysesystem nach einem der Ansprüche 1 bis 6, dadurch gekennzeichnet, daß der Sender (22) eine Infrarot-Leuchtdiode und der Empfänger (24) ein infrarotempfindlicher Fototransistor ist, die jeweils mit einer integrierten Strahlungsbündelungseinrichtung (32) versehen sind.

## Claims

1. Test strip analysis system consisting of a test strip and a test strip evaluation apparatus which includes a device for the optical recognition of a coding on the test strip, whereby the test strip is positively guidably movable in the evaluation apparatus relative to the reading device for the coding and the reading device has a light source as transmitter and a light-sensitive element as receiver, characterised in that

the test strip carries a bar code with an information density of more than 10 bit per cm.,

the transmitter (22) and the receiver (24) are placed on the same side of the test strip,

between test strip (10) and the transmitter (22) and between test strip (10) and receiver (24) is present a common cylindrical lens (28), the cylindrical axis (A) of which runs substantially parallel to the test surface (16) carrying the coding and to the code bars (20),

between the cylindrical axis (A) and the transmitter (22), as well as between the cylindrical lens (28) and the receiver (24), is, in each case, present an aperture slit,

both aperture slits (30) and the receiver (24) lie in the plane defined by the cylindrical axis (A) and the transmitter (22),

the distance between the cylindrical axis (A) and the test strip surface (16) is approximately so chosen that the focus line of the lens (28) lies about in the test strip surface (16), and the evaluation apparatus has a memory into which is transferred the information coded on the strip code.

2. Test strip analysis system according to claim 1, characterised in that two aperture slits are about the same distance from the cylindrical axis and have about the same width.

3. Test strip analysis system according to one of claims 1 or 2, characterised in that the transmitter (22), the receiver (24) and the aperture slits (26, 30) lie in a plane vertical to the test strip surface (16) symmetrically on both sides of the test strip middle plane.

4. Test strip analysis system according to one of claims 1 to 3, characterised in that the cylindrical lens (28) is formed as circular cylinder with a diameter of less than 2 mm.

5. Test strip analysis system according to claim 4, characterised in that the lens diameter amounts to a maximum of 1 mm.

6. Test strip analysis system according to one of claims 1 to 5, characterised in that the distance between the aperture (26) and the cylindrical lens (28) is at least about ten times as great as the focal length of the lens (28).

7. Test strip analysis system according to one of claims 1 to 6, characterised in that the transmitter (22) is an infra-red light diode and the receiver (24) an infra-red-sensitive phototransistor which, in each case, are provided with an integrated beam-concentrating device (32).

## Revendications

1. Système d'analyse de bandes de test constitué par des bandes de test et par un dispositif d'analyse des bandes de test et comprenant un dispositif de reconnaissance optique d'un code sur la bande de test, la bande de test pouvant être déplacée dans le dispositif d'analyse par rapport au dispositif assurant la lecture du code et le dispositif de lecture comportant une source lumineuse en tant qu'émetteur et un élément photosensible en tant que récepteur, caractérisé en ce que

les bandes de test portent un code de barres ayant une densité d'information de plus de 10 bits par cm, en ce que

l'émetteur (22) et le récepteur (24) sont placés du même côté de la bande de test qu'entre

la bande de test (10) et l'émetteur (22) et entre la bande de test (10) et le récepteur (24) est placée une lentille cylindrique commune (28) dont l'axe de cylindre (A) est essentiellement parallèle à la surface d'essais (16) qui porte le code et aux barres de code (10), en ce qu'entre

l'axe de cylindre (A) et l'émetteur (22) ainsi qu'entre la lentille cylindrique (28) et le récepteur (24) sont intercalés un ou des diaphragmes à fente, en ce que

la fente (30) du ou des diaphragmes et le récepteur (24) se trouve dans le plan défini par l'axe cylindrique (A) et l'émetteur (22), en ce que

la distance comprise entre l'axe de cylindre (A) et la surface de bandes de test (16) est approximativement choisie de telle manière que la ligne focale de la lentille (28) se trouve à peu près sur la surface (16) de la bande test et en ce que

le dispositif d'analyse comporte une mémoire dans laquelle les informations codées sur le code de barres sont enregistrées.

2. Système d'analyse de bandes de test selon la revendication 1, caractérisé en ce que les deux fentes de diaphragmes sont situées à des distances à peu près égales de la lentille cylindrique et elles ont à peu près la même largeur.

3. Système d'analyse de bandes de test selon l'une des revendications 1 ou 2, caractérisé en ce que l'émetteur (22), le récepteur (24) et les diaphragmes à fente (26, 30) sont disposés symétriquement des deux côtés du plan médian de la bande de test dans un plan perpendiculaire à la surface (16) de la bande de test.

4. Système d'analyse de bandes de test selon l'une des revendications 1 à 3, caractérisé en ce que la lentille cylindrique (28) est un cylindre de section circulaire ayant un diamètre de moins de 2 mm.

5. Système d'analyse de bandes de test selon la revendication 4, caractérisé en ce que le diamètre de la lentille est au maximum de 1 mm.

6. Système d'analyse de bandes de test selon l'une des revendications 1 à 5, caractérisé en ce que la distance entre le diaphragme (26) et la lentille cylindrique (28) est au moins dix fois plus grande environ que la distance focale de la lentille.

7. Système d'analyse de bandes de test selon l'une des revendications 1 à 6, caractérisé en ce que l'émetteur (22) est une diode lumineuse à infra-rouge, en ce que le récepteur (24) est un transistor sensible à la radiation infra-rouge et en ce qu'ils comportent tous deux un dispositif intégré (32) assurant la formation d'un faisceau de rayons.

FIG. 1

FIG. 2